# EUROPEAN PATENT APPLICATION

(11) **EP 2 389 935 A1**
(43) Date of publication of application: **30.11.2011**
(21) Application number: 11167335.6
(22) Date of filing: 24.05.2011
(51) Int. Cl.: A61K 31/197, A61K 9/00, A61K 9/08, A61K 47/36

(54) **Controlled-Release oral Solution Formulations of Pregabalin**

(30) Priority: 25.05.2010 TR 201004139; 25.06.2010 TR 201005145; 29.06.2010 TR 201005241
(71) Applicant: Sanovel Ilac Sanayi ve Ticaret A.S., 34398 Istanbul (TR)
(72) Inventor: Cifter, Ümit, 34398, Istanbul (TR); Türkyilmaz, Ali, 34398, Istanbul (TR); Akalin, Nur Pehlivan, 34398, Istanbul (TR)
(74) Representative: Sevinç, Erkan

(57) **Abstract**

The present invention relates to a controlled-release oral solution formulation, which gels in the stomach, comprising pregabalin or a pharmaceutically acceptable salt of pregabalin and a gelling agent.

## Description

### Field of Invention

The present invention relates to pharmaceutical compositions of pregabalin or a pharmaceutically acceptable salt thereof. The present invention more particularly relates to a stable controlled-release solution formulation of pregabalin, orally-administered, with a release profile of desired efficiency.

### Background of Invention

Pregabalin is an analog of gamma-aminobutyric acid (GABA). Its chemical designation is (S)-3-(aminomethyl)-5-methyl hexanoic acid, with the chemical structure illustrated below in Formula 1.

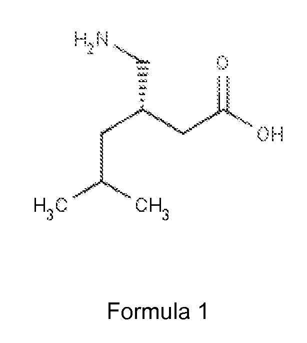

It is known that pregabalin binds to the auxiliary subunit of voltage-sensitive calcium channels in the central nervous system, thereby replacing [3H]-gabapentin. It also reduces the release of many neurotransmitters, including glutamate, noradrenaline, and the substance P. It is used for the treatment of epilepsy, simple or complex partial convulsion, either accompanied or not by secondary generalized convulsions, and of neuropathic pain. Various formulations of pregabalin have been developed. It is generally provided in the form of conventional capsule formulations. Posology requires such formulations to be administered twice or thrice daily.

Various applications can be found in relation to pregabalin in the patent literature.

Patent application W02008008120, for instance, discloses a solid dosage form comprising a compacted fill material containing at least one active agent and at least one of a disintegrating agent and wetting agent.

Patent application W02007052125 claims a pharmaceutical composition containing pregabalin or a pharmaceutically acceptable complex, salt, solvate, or hydrate thereof, and excipients such as a matrix forming agent and a swelling agent.

Patent application W02008140459, in turn, discloses a solid dosage form comprising a compacted fill material having a pressure-sensitive multi-particulate and at least one buffering agent. Said multi-particulate and/or buffering agent comprises at least one active agent and display(s) a weight loss less than 1% in 30 minutes according to the friability test USP 29 test # 1216. The compacted fill material has a density of at least 0.5 g/ml and a tensile strength of less than 0.9 MPa.

Patent application W02008140460, on the other hand, claims a solid dosage form comprising a compacted fill material including at least one active agent. The solid dosage form displays a weight loss of less than 1% in 30 minutes in accordance with the friability test USP 29 test # 1216. Compacted fill material particles contain at least one active agent in the matrix and provide a controlled release of the active agent.

Patent application W02008128775 claims a solid pharmaceutical composition comprising pregabalin as an active agent, together with one or more excipients. Said composition is free from saccharides and comprises no further amino acids.

Patent application W02009066325 provides a controlled release formulation comprising pregabalin or a pharmaceutically acceptable salt thereof, a hydrophobic release agent, and an excipient.

Stability-related problems do occur in a plurality of active agents, including pregabalin, under the influence of ambient and physical conditions. As disclosed in patent W09959573, pregabalin, an amino acid derivative, undergoes cyclization and converts into a lactam form, even if normal storage conditions are provided. This is not desirable for the formulation.

Pregabalin is an active agent with quite good solubility and dissolution rate. This fact leads to fluctuations in the release profile of controlled-release formulations aimed to be developed. Such fluctuations, in turn, brings about imbalances in the blood plasma levels of active agent, and resultantly, undesired effects are produced on the subject.

Another problematic situation for pregabalin, in fact, is its absorption in the gastrointestinal tract. It has been observed that pregabalin absorption increases from small intestine towards large intestine, and becomes poor beyond the hepatic flexure. Conventional tablets are transferred to the hepatic flexure in about 6 hours or less, on average, thereafter loosing efficiency due to poor absorption in the remaining parts of the intestine.

In result, the aforesaid drawbacks require a novelty in the art of pregabalin formulations with antiepileptic, analgesic, and anxiolytic activities.

### Object and Brief Description of Invention

The present invention relates to a controlled-release pregabalin formulation, gelling in the stomach and floating in gastric juice, thereby eliminating all aforesaid problems and bringing additional advantages to the relevant prior art.

Accordingly, the main object of the present invention is to obtain a stable formulation with antiepileptic, analgesic, and anxiolytic activities.

Another object of the present invention is to provide a pregabalin formulation administered orally once or twice per day.

A further object of the present invention is to ensure a high drug-absorption by retarding the advancement of formulation through the gastrointestinal tract, thanks to a pregabalin formulation that gels in the stomach and floats in gastric juice.

Yet a further object of the present invention is to provide a stable formulation by preventing pregabalin used in the subject formulation from converting into the lactam form via cyclization.

Still a further object of the present invention is to embody a controlled-release formulation with uniform release profile.

A controlled-release oral solution formulation which gels in the stomach has been developed to carry out any objects, referred to above and to emerge from the following detailed description.

According to a preferred embodiment of the present invention, said novelty is carried out with pregabalin or a pharmaceutically acceptable salt thereof and a gelling agent. Preferred gelling agents comprise at least one of calcium or sodium alginate or guar gum, or a properly-proportioned mixture thereof.

In a preferred embodiment according to the present invention, the amount of said gelling agent is not more than 15% by weight.

In a preferred embodiment according to the present invention, also polycarbophil is included for use as a controlled-release agent.

In a preferred embodiment according to the present invention, the proportion of polycarbophil to the total tablet weight is 0.01 to 2%.

In a preferred embodiment according to the present invention, a buffering agent is also included for use an excipient.

In a preferred embodiment according to the present invention, said buffering agent comprises at least one of sodium citrate, calcium carbonate, or a mixture thereof.

In a preferred embodiment of the present invention, said gel formulation is floatable in gastric juice.

Another preferred embodiment according to the present invention provides a method for preparing said pharmaceutical formulation, this method comprising the steps of
a. dissolving sodium citrate in deionized water and adding gelling agent into the resulting mixture,
b. mixing the resulting mixture with heating to 90°C, then cooling it back to 40°C,
c. adding calcium carbonate, pregabalin (the active ingredient), and other excipients (flavoring agent, sweetener, water, preservative) into the solution and mixing the same.

In another preferred embodiment according to the present invention, said pharmaceutical formulation is consisting of
a. pregabalin or a pharmaceutically acceptable salt thereof at 25-75% by weight,
b. guar gum, sodium alginate or calcium alginate, or a properly-proportioned mixture thereof at 0.1-15% by weight,
c. buffering agent at 0.1 to 10% by weight.

### Detailed Description of Invention

### Example 1:

| Ingredient | **amount** % |
|---|---|
| pregabalin | 25 - 75 |
| guar gum, sodium alginate or calcium alginate, or a properly-proportioned mixture thereof | 1 - 15 |
| sodium citrate | 0.75 - 2 |
| calcium carbonate | 0.75 - 2 |
| flavoring agent, sweetener, preservative | 0,05 - 6 |

### Example 2:

| **Ingredient** | **amount** % |
|---|---|
| pregabalin | 25 - 75 |
| guar gum, sodium alginate or calcium alginate, or a properly-proportioned mixture thereof | 1 - 15 |
| polycarbophil | 0,5 - 2 |
| sodium citrate | 0.75 - 2 |
| calcium carbonate | 0.75 - 2 |
| flavoring agent, sweetener, preservative | 0,05 - 5 |

Sodium citrate in dissolved in deionized water and gelling agent is added into the resulting solution. This mixture is mixed under heating to 90°C, it is then cooled down to 40°C. Then calcium carbonate, pregabalin, and other excipients (flavoring agent, sweetener, water, preservative) are added to the solution and the latter is mixed.

This invention has surprisingly provided a controlled-release pregabalin solution formulation, which gels in the stomach and floats in gastric juice, as well as is stable and has a desired release profile. The formulation according to the present invention swells upon contact with gastric juice, thereby lowering its density and floating in gastric juice. Thanks to this fact, the advancement of the formulation through the gastrointestinal tract is retarded. Thus, the advancement of the pregabalin-containing formulation through the gastrointestinal tract is delayed and its absorption is made to occur at a site in which adsorption takes place more efficiently. An ideal absorption of pregabalin occurs only at a certain site of small intestine. Retaining the drug at an efficient absorption site enhances its bioavailability.

It is possible to make various controlled-release formulations containing pregabalin. For instance, it is possible to develop formulations which are retained in the stomach so that their advancement is delayed, or which are solid tablet out of the body and gel in the stomach with a volume increase. These systems, for instance, can float within stomach due to their low densities, or may be mucoadhesive and adhered at the stomach. Various excipients can be used for this purpose, such as alginates (salts thereof), gums, oil, gelling agents.

The present invention is used for treating epilepsy, pain, anxiety, diabetic neuropathy, neuropathic pain, partial seizure, social phobia, postherpetic neuralgia.

It is further possible to use the following additional excipients in the formulation.

The pharmaceutical compositions according to the present invention may also comprise one or more pharmaceutically acceptable excipients. Such pharmaceutically acceptable excipients include, but are not restricted to gelling agents, fillers, binders, glidants, lubricants, disintegrants, surface active agents, preservatives, coating agents etc., and the mixtures thereof.

Suitable gelling agents include, but are not restricted to aluminum silicate, calcium silicate, carbomer, croscarmellose sodium carrageen, chitosan, gelatin, glyceryl monooleate, glyceryl palmitostearate, hydroxyethyl cellulose, microcrystalline cellulose and croscarmellose sodium mixture, pectin, polyethylene alkyl ether, polyethylene glycol, polyethylene oxide, polymethyl methacrylate, propylene carbonate, sodium ascorbate, gellan gum, and mixtures thereof.

Suitable preservatives include, but are not restricted to, at least one or a mixture of methyl paraben and propyl paraben and salts thereof (e.g. sodium or potassium salts), sodium benzoate, citric acid, benzoik acid, butylated hydroxytoluene and butylated hydroxyanisole.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

The present invention is hereby disclosed by referring to an exemplary embodiment hereinabove. Whilst this exemplary embodiment does not restrict the object of the present invention, the latter must be assessed under the light of the foregoing detailed description.

## Claims

1. A controlled-release oral solution formulation, which gels in the stomach, comprising pregabalin or a pharmaceutically acceptable salt of pregabalin and a gelling agent.

2. The pharmaceutical formulation according to Claim 1, wherein the amount of said gelling agent is not more than 15% by weight.

3. The pharmaceutical formulation according to any of the preceding claims, wherein said gelling agent comprises at least one of calcium or sodium alginate or guar gum, or a properly-proportioned mixture thereof.

4. The pharmaceutical formulation according to any of the preceding claims, further comprising polycarbophil.

5. The pharmaceutical formulation according to any of the preceding claims, wherein the proportion of polycarbophil to the total weight is 0.01 to 2%.

6. The pharmaceutical formulation according to any of the preceding claims, further comprising a buffering agent as an excipient.

7. The pharmaceutical formulation according to any of the preceding claims, wherein said buffering agent comprises at least one of sodium citrate, calcium carbonate, or a mixture thereof.

8. A method for preparing a pharmaceutical formulation according to any of the preceding claims, comprising the steps of
a. dissolving sodium citrate in deionized water and adding gelling agent into the resulting mixture,
b. mixing the resulting mixture under heating to 90°C, then cooling it down to 40°C,
c. adding calcium carbonate, pregabalin (the active agent), and other excipients (flavoring agent, sweetener, water, preservative) into the solution and mixing the same.

9. The pharmaceutical formulation according to any of the preceding claims, consisting of
a. pregabalin or a pharmaceutically acceptable salt thereof at 25-75% by weight,
b. guar gum, sodium alginate or calcium alginate, or a properly-proportioned mixture thereof at 0.1-15% by weight,
c. buffering agent at 0.1 to 10% by weight.

10. A pharmaceutical formulation according to any of the preceding claims, for preventing or treating epilepsy, pain, anxiety, diabetic neuropathy, neuropathic pain, postherpetic neuralgia in mammalians, but particularly in humans.

11. The pharmaceutical formulation according to any of the preceding claims, said gel being floatable in gastric juice.
